# EUROPÄISCHE PATENTSCHRIFT

(11) **EP 1 394 264 B1**
(45) Veröffentlichungstag und Bekanntmachung des Hinweises auf die Patenterteilung: **03.11.2004**
(21) Anmeldenummer: 03016728.2
(22) Anmeldetag: 22.07.2003
(51) Int. Cl.: C12Q 1/04

(54) **Gammasterilisierbare Kulturmedien auf der Basis von Caseinsojapeptonagar zum Nachweis von Mikroorganismen in wasserstroffperoxidhaltiger Luft oder auf wasserstoffperoxidhaltigen Oberflächen**
Gamma-sterilisable culture media on the basis of casein soja peptone agar for determining the presence of microorganismus in gaseous environment or on hydrogen peroxide containing substances
Milieux de cultures gamma stérilisables à base d'agar composé de peptones, de soja et de caséine pour la détection de micro-organismes dans de l'air ou sur des surfaces contenant du péroxyde d'hydrogène

(30) Priorität: 23.07.2002 DE 10233346
(43) Veröffentlichungstag der Anmeldung: 03.03.2004
(73) Patentinhaber: BIOTEST AG, D-63303 Dreieich (DE)
(72) Erfinder: Horn, Jürgen, Dr., 63329 Egelsbach (DE)
(74) Vertreter: Strych, Werner Maximilian Josef, Dr.

(56) Entgegenhaltungen:
- EP-A- 1 329 515
- REIDMILLER JEFFREY S ET AL: "Characterization of UV-peroxide killing of bacterial spores." JOURNAL OF FOOD PROTECTION, Bd. 66, Nr. 7, Juli 2003 (2003-07), Seiten 1233-1240, XP009020105 ISSN: 0362-028X (ISSN print)
- ARANA INES ET AL: "Role of hydrogen peroxide in loss of culturability mediated by visible light in Escherichia coli in a freshwater ecosystem" APPLIED AND ENVIRONMENTAL MICROBIOLOGY, Bd. 58, Nr. 12, 1992, Seiten 3903-3907, XP009020101 ISSN: 0099-2240
- FROLANDER F ET AL: "BACTERICIDAL EFFECT OF ANAEROBIC BROTH EXPOSED TO ATMOSPHERIC OXYGEN TESTED ON PEPTOSTREPTOCOCCUS-ANAEROBIUS" JOURNAL OF CLINICAL MICROBIOLOGY, Bd. 6, Nr. 2, 1977, Seiten 117-123, XP009020100 ISSN: 0095-1137
- MCDONALD L C ET AL: "ENHANCED RECOVERY OF INJURED ESCHERICHIA-COLI BY COMPOUNDS THAT DEGRADE HYDROGEN PER OXIDE OR BLOCK ITS FORMATION" APPLIED AND ENVIRONMENTAL MICROBIOLOGY, Bd. 45, Nr. 2, 1983, Seiten 360-365, XP009020103 ISSN: 0099-2240
- DATABASE BIOSIS [Online] BIOSCIENCES INFORMATION SERVICE, PHILADELPHIA, PA, US; 1990 DUBININA G A ET AL: "HYDROGEN PEROXIDE PRODUCTION BY BEGGIATOA-LEPTOMITIFORMIS" Database accession no. PREV199191054508 XP002259708 & MIKROBIOLOGIYA, Bd. 59, Nr. 3, 1990, Seiten 425-431, ISSN: 0026-3656

## Beschreibung

Gegenstand der Erfindung ist ein gammasterilisierbares Kulturmedium zum Nachweis von Mikroorganismen, wie Bakterien, Hefen und Pilzen, in wasserstoffperoxidhaltiger Luft oder auf wasserstoffperoxidhaltigen Oberflächen mit den Markmalen des Patentspruchs 1. Bevorzugte ergänzende Ausgestaltungen sind Gegenstand der Unteransprüche.

Wasserstoffperoxid wird zum Begasen von Isolatoren oder ganzen Räumen benutzt, um darin möglicherweise enthaltene Mikroorganismen abzutöten. Das gasförmige Wasserstoffperoxid kondensiert als 30 % bis 35%ige gesättigte Lösung auf den begasten Oberflächen. Vor dem Start von Produktionsvorgängen, Qualitätskontrolluntersuchungen auf Sterilität oder sonstigen Arbeiten in diesen begasten Reinraumbereichen werden diese steril belüftet, wonach Konzentrationen von 0,3 bis 6ppm, in der Regel unter 1ppm, Keime in der Luft verbleiben. Die Untersuchungen auf solche möglicherweise noch vorhandene Luftkeime erfolgt mit Luftkeimsammlern nach dem Impaktions- oder Rotationsprinzip, wobei die keimhaltigen Partikel auf Agaroberflächen abgeschieden werden.

Überraschenderweise konzentrieren sich die geringen Mengen Wasserstoffperoxiddämpfe beim Vorgang des Sammelns von 1000 Litern Luft in Caseinsojapeptonagar (gem. United States Pharmakopoeae, 8^{th} Supplement, USP-NF, <1116>, 4426-4431) auf Konzentrationen von über 100ppm im Agar. Sporen werden schon durch Wasserstoffperoxid-Konzentrationen von 10ppm gehemmt und vegetative Zellen und Mikroorganismen bereits durch noch deutlich niedrigere Konzentration an Wasserstoffperoxid. Dies beeinträchtigt die Nachweismöglichkeit von noch vorhandenen Mikroorganismen in erheblichem Maß.

Normale Agarmedien weisen eine Kontaminationsrate von etwa 0,1 % auf (1 unsterile Einheit unter 1000) und genügen damit nicht dem Begriff von Sterilität, welcher nur 1 unsterile Einheit unter 10⁶ (1 Mio.) zulässt. Da mit den zur Untersuchung verwendeten Medien Reinräume untersucht werden, welche durch Begasung vorher keimfrei gemacht wurden, möchte man mit den Agarmaterialien zur Testung auf Keimfreiheit keine Keime einschleppen, erwartet also sterile Medien.

Bekannt sind für Nachweise der genannten Art Medien wie Baird-Parker Medium (Journ. Applied Bacteriology 25:12, 1962, Baird-Parker) mit 1 % Natriumpyruvat (Natriumsalz der Brenztraubensäure oder 2-Oxopropionsäure), die unter anderem für die Isolierung von Staphylokokkus aureus nach Hitzeschädigung oder Lagerung von gefrorenen oder getrockneten vegetativen Zellen geeignet sind. Der Nachteil von Baird-Parker-Agar ist die geringe Haltbarkeit des fertigen Agarmediums.

Der Zusatz von Katalase zu diesem und anderen Medien zur Verbesserung des Wachstums von Bakterien aus der Luft ist ebenfalls bekannt (Journ. Applied And Environmental Microbiology 57: 2775-2776, 1991, Balkumar Marthi). Katalase zersetzt Wasserstoffperoxid in Wasser und Sauerstoff. Katalase wird jedoch bei 55° C inaktiviert, was die großtechnische Abfüllung von Agar bei deutlich unter 55° C voraussetzt. Dies ist jedoch wegen Gelieren des Agars bei Temperaturen um 50° C kaum durchführbar. Außerdem verursacht der aus Wasserstoffperoxid entstehende Sauerstoff Blasen und Risse im Agar, die das Erkennen von Kolonien, die auf dem Agar wachsen, außerordentlich erschwert.

Weiter ist D/E-Agar bekannt, welcher ein breites Spektrum an antiseptischen und desinfizierenden Chemikalien einschließlich quaternärer Ammoniumverbindungen, Phenol, Jodund Chlorverbindungen, Quecksilber (Merthiolat), Formaldehyd und Glutaraldehyd neutralisiert (Difco Handbuch, D/E Agar). Eine Neutralisation von Wasserstoffperoxid ist im Difco Handbuch nicht beschrieben. Der Nachteil von D/E-Agar ist die geringe Haltbarkeitsdauer des fertigen Agarmediums von nur 2½ Monaten sowie die Veränderungen im Medium bei Strahlungsdosen von 16-25 kgray, die zur zuverlässigen Gammasterilisierung notwendig sind.

Außerdem ist D/E-Agar nicht sehr pH stabil und hat mit einem pH von 7,6 ± 0,2 schon einen sehr hohen pH Wert. Bei einem Abdriften zu noch höheren pH Werten wie 8,0 (nur 0,2 über dem oberen Bereich) kommt es bereits zu deutlichen Hemmungen von nachzuweisenden Keimen.

Erstaunlicherweise wurde nun gefunden, dass Agarmedien auf der Basis von Caseinsojapeptonagar Wasserstoffperoxid in Konzentrationen, wie sie beim Luftkeimsammeln in Isolatoren mit Wasserstoffperoxid Restgehalt auftreten, neutralisiert, wenn ihnen 2 - 10 Gew.% Natriumthioglykolat, 5 - 20 Gew.% Natriumbisulfit und 10 - 30 Gew.% Natriumthiosulfat, jeweils bezogen auf das Agar, zugesetzt werden. Vorzugsweise wird als Caseinsojapeptonagar das Microbial Content Test Agar (MCT Agar; Difco 0553-07-4) eingesetzt, welcher aus Caseinsojapepton mit Zusatz von Sorbitanmonooleat = Tween 80® und Lecithin besteht. Durch Pufferung im pH Bereich von 7,1 bis 7,5 sind diese Medien außerdem pH-stabil.

Die übliche Pufferung mit Phosphatpuffer allein führt zu Ausfällungen, sodass das Medium schlechte Wachstumseigenschaften hat. Dies kann bekanntermaßen durch die Pufferung mit MOPS (Morpholinopropansulfonicacid) vermieden werden. MOPS ist jedoch sehr teuer. Überraschenderweise hat es sich jedoch gezeigt, dass ein teilweiser Ersatz von Phosphat durch MOPS möglich ist, ohne dass es zu wachstumsmindernden Ausfällungen kommt. Vorzugsweise werden von der Gesamtpuffermenge 20-50% als MOPS verwendet, wobei MOPS zuerst zugegeben wird und danach langsam der Rest von 50-80% des Gesamtpuffergehaltes als Phosphatpuffer.

Die üblicherweise zugesetzten pH-Indikatoren Bromkresolpurpur und Bromthymolblau werden durch Gammabestrahlung mit 16-25 K Gray zerstört mit der Folge eines grauen Erscheinungsbildes des Agar, welches die Auswertung von weißen bis grauen Kolonien wesentlich erschwert. Dies lässt sich erstaunlicherweise durch den Zusatz von Polyvinylpyrolidon in Mengen von 10 - 50 Gew.%, vorzugsweise 30 - 45 Gew.%, bezogen auf das Agar, verhindern. Die blauviolette bis blaugrüne Farbe bleibt dann erhalten, was die Auswertung und Überprüfung auf gewachsene Kolonien wesentlich erleichtert.

Überraschenderweise kann die wasserstoffperoxidneutralisierende Wirkung der erfindungsgemäßen Nährmedien durch Zusatz von 0,05 bis 0,25 % Pyruvat verstärkt werden. Diese im Vergleich mit Baird-Parker-Medium wesentlich geringere Konzentrationen ist wegen des hohen Preises von Natriumpyruvat wirtschaftlich von Bedeutung.

Erfindungsgemäße Medien, gepuffert im Bereich von pH 6,8 bis 7,4, mit den genannten pH-Indikatoren und Zusatz von Natriumpyruvat und Polyvinylpyrolidon sind 6 Monate stabil, was die Lagerung und den Versand wesentlich erleichtert und dem Kunden noch eine lange Verwendungsdauer garantiert. Weiter sind diese Medien in der Lage, 2 %ige H₂O₂-Lösungen, die direkt aufgetragen werden, zu neutralisieren und anschließendes Wachstum von Mikroorganismen zu erlauben. Demgegenüber erlaubt beispielsweise normaler Sojacaseinpeptonagar bereits nach einer Exposition mit nur 0,02 % Wasserstoffperoxid kein Keimwachstum mehr.

Weiter kann zum besseren Anwachsen von Keimen, welche durch Austrocknung (in der Luft oder auf Oberflächen) geschädigt wurden, Betain, Glycin, Cystin, Prolin und Asparagin zugesetzt werden.

Die folgenden Beispiele erläutern die Erfindung im einzelnen.

### Beispiel 1 Medium mit Farbindikator zum Abklatsch auf H₂O₂-haltigen Oberflächen

| | |
|---|---|
| Grundmedium Microbial Content Test Agar (Difco 0553-07-4 = MCT Agar) | 23 g |
| Agar-Agar (bestehend aus Caseinsojapepton, Kochsalz, Lecithin, Sorbitanmono-oleat und Agar) | 12 g |
| Polyvinylpyrolidon (PVP 360)Betain (Sigma B3501)0,03 | 10 g |
| Betain (Sigma B3501) | 0,03 g |
| L-Glycin (Merck 104201) | 0,05 g |
| L-Cystin (Merck 1028136) | 0,025 g |
| L-Prolin (Merck 107434) | 0,025 g |
| Benztraubensäure, Na-Salz (Merck 106619 ) =Natriumpyruvat | 0,25 g |
| L-Asparagin (Merck 101565) | 0,025 g |
| Glucose (Merck 107074) | 2,5 g |
| Natriumthioglycolat (Sigma T0632) | 1,0 g |
| Natriumdisulfit (Merck 106528) | 2,5 g |
| Natriumthiosulfat (Merck 106516) | 6,0 g |
| Bromkresolpurpur (Merck 103025) | 0,025 g |
| Bromthymolblau (Merck 103026) | 0,025 g |
| Aqua dest | ad 1 Liter |
| pH auf 7,3 ± 0,2 einstellen, 15 min. bei 121° C autoklavieren und nach dem Abkühlen sterilfiltriert zugeben: Hefeextrakt | 2,5 ml |
| (Marcor, 10g Hefeextrakt in 100 ml VE-Wasser kalt eingerührt und sterilfiltriert) | |
| Phosphat Puffer pH 7,3 1 Molare Lösung | 20 ml |
| MOPS Puffer pH 7,3 4 Molare Lösung | 6 ml |
| (Sigma M1254, Morpholinopropansulfonicacid) | |
| (zuerst MOPS zugeben, danach langsam Phosphat) | |
| L-Ascorbinsäure (Na-Salz Sigma A7631, 1 g in 2 ml VE Wasser | 0,5 ml |

### Beispiel 2 Medium ohne Farbindikator zum Nachweis von Luftkeimen in H₂O₂-haltiger Isolatorluft

| | |
|---|---|
| Grundmedium Microbial Content Test Agar (Difco 0553-07-4) | 23 g |
| Agar-Agar | 12 g |
| Betain (Sigma B3501) | 0,03 g |
| L-Glycin (Merck 104201) | 0,05 g |
| L-Cystin (Merck 1028136) | 0,025 g |
| L-Prolin (Merck 107434) | 0,025 g |
| Benztraubensäure, Na-Salz (Merck 106619) =Natriumpyruvat | 0,25 g |
| L-Asparagin (Merck 101565) | 0,025 g |
| Glucose (Merck 107074) | 2,5 g |
| Natriumthioglycolat (Sigma T0632) | 1,0 g |
| Natriumdisulfit (Merck 106528) | 2,5g |
| Natriumthiosulfat (Merck 106516) | 6,0 g |
| Aqua dest | ad 1 Liter |
| pH auf 7,3 ± 0,2 einstellen, 15 min. bei 121° C autoklavieren und nach dem Abkühlen sterilfiltriert zugeben: | |
| Hefeextrakt | 2,5 ml |
| (Marcor, 10g Hefeextrakt in 100 ml VE-Wasser kalt eingerührt und sterilfiltriert) | |
| Phosphat Puffer pH 7,3 1 Molare Lösung | 20 ml |
| MOPS Puffer pH 7,3 4 Molare Lösung | 6 ml |
| (Sigma M1254, Morpholinopropansulfonicacid) | |
| (zuerst MOPS zugeben, danach langsam Phosphat) | |
| L-Ascorbinsäure (Na-Salz Sigma A7631, 1 g in 2 ml VE Wasser | 0,5 ml |
| In Agarstreifen für Luftkeimsammelgerät gießen und γ-sterilisieren (Dosis 16 - 25 K Gy) | |

### Beispiel 3 Microbial Content Test Agar

Difco ohne Zusätze

### Beispiel 4 Soybean Casein Digest Agar

Difco mit 1 % (10 g/L) zusätzlich Natriumpyruvat

### Beispiel 5 D/E Agar

Difco ohne Zusätze

Alle 5 Agar-Sorten werden zur Austestung ihrer Kapazität zur Neutralisation von H₂O₂ mit 100 Mikroliter von H₂O₂-haltigen Lösungen mit 10ppm 0,02 % H₂O₂, 0,5 % H₂O₂, 1 % H₂O₂ und 2 % (20000ppm) H₂O₂ beaufschlagt. Danach wird die H₂O₂ Konzentration auf der Agaroberfläche mit Peroxid Teststreifen (Merck) gemessen (Tab. 1). Während der erfindungsgemäße Agar noch 2 % (20000ppm) H₂O₂ neutralisiert, ist das Grundmedium MCT allein nicht in der Lage 10ppm komplett zu neutralisieren. Aus der Literatur bekannte Agarsorten (Beispiel 4 und 5) können bereits 0,5 % H₂O₂ nicht mehr vollständig neutralisieren.

Nach Exposition mit H₂O₂ wird mit Beimpfung von Staphylococcus aureus ATCC 6538 mit 10-100 koloniebildenden Einheiten das mögliche Wachstum nach H₂O₂ Exposition untersucht (Tab. 2).

Die erfindungsgemäßen Agarzusätze nach Beispiel 1 und 2 erlauben das Keimwachstum auch nach Exposition mit hohen H₂O₂-Mengen, während der eingesetzte Grundagar bereits durch 10ppm H₂O₂ deutliche Hemmungen beim Wachstum zeigt. Die literaturmäßig bekannten Varianten mit Pyruvatzusatz allein oder D/E Agar in bekannter Form neutralisieren etwas mehr H₂O₂, zeigen jedoch auch bereits ab 0,5 % H₂O₂ sehr deutliche Wachstumshemmungen.

**Tabelle 1:**

| *H*_{*2*}*O*_{*2*} *Konzentration im Agar nach Aufbringen von 100 Mikrolitern H*_{*2*}*O*_{*2*} *Lösungen* | | | | | |
|---|---|---|---|---|---|
| **Konzentration** **der** **aufgebrachten** **H**_{**2**}**O**_{**2**} **Lösung** | **Agar** **Beispiel 1** **(Erfindung)** | **Agar** **Beispiel 2** **(Erfindung)** | **MCT Agar** **Beispiel 3** **(Standard-vergleich)** | **Soybean** **Casein Digest** **mit 1 %** **Pyruvat** **Beispiel 4** **(Literatur)** | **D/E Agar** **Beispiel 5** **(Vergleich)** |
| 10ppm | 0ppm | 0ppm | 1-2ppm | 0ppm | 0ppm |
| 0,02 % | 0ppm | 0ppm | 30ppm | 0ppm | 0ppm |
| 0,5 % | 0ppm | 0ppm | > 100ppm | 2-5ppm | 2-5ppm |
| 1,0 % | 0ppm | 0ppm | > 100pmm | 5,10ppm | 10ppm |
| 2,0 % (=20000ppm) | 0ppm | 0ppm | | 20-30ppm | 30ppm |

**Tabelle 2:**

| *Wachstum von Staph. aureus 6538 nach H2O2 Exposition - Inokulum 10-100* *kolonie-bildende Einheiten (KBE) pro Agaroberfläche (Petrischale Agarstreifen* *Contact Slide)* | | | | | |
|---|---|---|---|---|---|
| **Konzentration** **der** **aufgebrachten** **H**_{**2**}**O**_{**2**} **Lösung** | **Agar** **Beispiel 1** **(Erfindung)** | **Agar** **Beispiel 2** **(Erfindung)** | **MCT Agar** **Beispiel 3** **(Standard-vergleich)** | **Soybean** **Casein Digest** **mit 1 %** **Pyruvat** **Beispiel 4** **(Literatur)** | **D/E Agar** **Beispiel 5** **(Vergleich)** |
| 0 = Kontrolle | 68 KBE | 73 KBE | 61 KBE | 71 KBE | 62 KBE |
| 10ppm | 63 KBE | 74 KBE | 18 KBE | 68 KBE | 73 KBE |
| 0,02 % | 71 KBE | 64 KBE | 0 KBE | 62 KBE | 65 KBE |
| 0,5% | 61 KBE | 59 KBE | 0 KBE | 12 KBE | 14 KBE |
| 1,0% | 65 KBE | 67 KBE | 0 KBE | 0 KBE | 0 KBE |
| 2,0 % (=200000ppm) | 69 KBE | 62 KBE | 0 KBE | 0 KBE | 0 KBE |

Alle 5 Agarsorten wurden in Agarstreifen für RCS Highflow Luftkeimsammelgeräte gegossen.

Danach wurden jeweils parallel Luftproben in einem Isolator mit vergleichsweise hoher H₂O₂ Restbelastung gesammelt sowie in einem über Nacht gelüfteten Isolator mit niedriger H₂O₂ Restbelastung. Zum Vergleich wurde jeweils unbelastete Luft aus einer Cleanbench im Reinraum gesammelt. Die Beimpfung mit einem Inokulum von 10-100 koloniebildenden Einheiten von Staph. aureus ATCC 6538 erfolgte jeweils direkt nach dem Luftkeimsammeln, um einer möglichen Reduktion des im Agar angesammelten H₂O₂ beim Stehen lassen zuvorzukommen. Damit sollte die H₂O₂ Belastung derjenigen entsprechen, die ein möglicher Luftkeim direkt beim Sammelvorgang hat. Man sieht in Tab. 3, dass ein normaler Standardagar das Keimwachstum nach Exposition mit normaler Luft ohne H₂O₂ erlaubt, jedoch nicht mehr in H₂O₂ belasteter Luft, unabhängig von der Konzentration. Die literaturbekannten Agarsorten 4 und 5 zeigen bereits deutliche Performanceschwächen in Isolator 1 bei höherer H₂O₂ Restkonzentration, während die erfindungsgemäßen Agarsorten nach Beispiel 1 und 2, wie nach den Ergenissen aus Tab. 1 zu erwarten war, auch höhere H₂O₂-Konzentrationen neutralisieren und ungehemmtes Wachstum erlauben. Die Ergebnisse sind in Tab. 3 zusammengestellt.

**Tabelle 3**

| *Luftkeimmessungen im Isolator. 1000 L H*_{*2*}*O*_{*2*}*-haltige Isolatorluft wird mit einem* *RCS Luftkeimsammler gesammelt und die Streifen danach mit S. aureus 6538* *beimpft. Vergleich unbelastet Cleanbench Luft.* | | | |
|---|---|---|---|
| **Agarsorte** | **1000 L Luft** **Isolator 1 hohe H**_{**2**}**O**_{**2**} **Restkonzentration** | **1000 L Luft** **Isolator 2 niedrige H**_{**2**}**O**_{**2**} **Restkonzentration** | **1000 L Cleanbench** **Luft ohne H**_{**2**}**O**_{**2**} **Restkonzentration** |
| Agar Beispiel 1 (Erfindung) | 86 | 92 | 83 |
| Agar Beispiel 2 (Erfindung) | 81 | 79 | 88 |
| MCT Agar Beispiel 3 (Standardvergleich) | 0 | 0 | 91 |
| Soybean Casein Digest mit 1 % Pyruvat Beispiel 4 (Literatur) | 11 | 74 | 83 |
| D/E Agar Beispiel 5 (Vergleich) | 7 | 92 | 94 |

Die Durchführung eines Abklatsches von H₂O₂ belasteten Oberflächen im Isolator in Abhängigkeit von der Lagerzeit des verwendeten Agars zeigt in Tab. 4 eindeutig die wesentlich bessere Stabilität der nutritiven Eigenschaften des erfindungsgemäßen Mediums nach Beispiel 1 gegenüber dem literaturbekannten D/E Standardagar. Bei Standard MCT Agar sind die nutritiven Eigenschaften zwar stabiler, jedoch auf etwas niedrigerem Niveau bei Staph. aureus. Bei dem anaeroben Keim C. sporogenes sieht man, dass die mangelnde H₂O₂-Neutralisierung von MCT Agar das Wachstum von Anaeroben nach H₂O₂ Exposition verhindert, während dies mit dem erfindungsgemäßen Agar noch ohne weiteres möglich ist. Bei anaeroben Keimen wird nach Beimpfung immer anaerob inkubiert.

Die Inkubationstemperatur für alle Bakterien wird nach USP bei 32,5° C ± 2,5° C eingestellt.

**Tabelle 4**

| *Wachstum von S. aureus 6538 und C. sporogenes nach Abklatsch von H*_{*2*}*O*_{*2*} *begasten Isolaforoberflächen* | | | | | | | | |
|---|---|---|---|---|---|---|---|---|
| Agarsorte Alter 4 Wo | Wachstum von | | Agarsorte Alter 3 Mon | Wachstum von | | Agarsorte Alter 6 Mon | Wachstum von | |
| | S. aureus | C. sporogenes | | S. aureus | C. sporog. | | S. aureus | C. sporog. |
| Agar Beispiel 1 (Erfindung) | 71 | 68 | Agar Beispiel 1 (Erfindung) | 88 | 92 | Agar Beispiel 1 (Erfindung) | 69 | 98 |
| MCT Agar Beispiel 3 (Standard-vergleich) | 58 | 0 | MCT Agar Beispiel 3 (Standard-vergleich) | 49 | 0 | MCT Agar Beispiel 3 (Standard- vergleich) | 52 | 0 |
| D/E Agar Beispiel 5 (Vergleich) | 68 | 16 | D/E Agar Beispiel 5 (Vergleich) | 23 | 0 | D/E Agar Beispiel 5 (Vergleich) | 0 | 0 |

In Tab. 5 wird das Ergebnis des Wachstums eines ganzen Keimspektrums nach Durchsatz von je 1000 Liter H₂O₂-haltiger Isolatorluft im Vergleich zu je 1000 Liter Luft aus einer Cleanbench ohne Isolator zusammengefasst. Das Wachstum von grampositiven Kokken (S. aureus), grampositiven sporenbildenden Stäbchen (B. subtilis), Anaeroben Sporenbildnern (C. sporogenes), gramnegativen Enterobacteriaceae (E. coli), gramnegativen Nonfermentem (P. aeruginosa), Hefen (C. albicans) und Pilzen (A. niger) wird geprüft. Auf MCT Agar und erfindungsgemäßem Agar wachsen alle Keime nach Exposition mit normaler Luft (Cleanbench Luft) gleich gut. Nach Exposition von H₂O₂-haltiger Isolatorluft wachsen auf dem erfindungsgemäßen Agar nach Beispiel 2 alle Keime in vergleichbarer Zahl, während auf MCT Agar nach Exposition mit H₂O₂-haltiger Isolatorluft mit allen Keimen keinerlei Wachstum mehr feststellbar ist.

**Tabelle 5**

| *Wachstum nach Sammeln von H*_{*2*}*O*_{*2*}*-haltiger Isolatorluft bzw. Cleanbench Luft* *ohne H*_{*2*}*O*_{*2*} | | | | |
|---|---|---|---|---|
| Keim | Agar Beispiel 2 | MCT Agar Beispiel 3 | Agar Beispiel 1 | MCT Agar Beispiel 3 |
| | 1000 L Isolatorluft | | 1000 L Cleanbench Luft | |
| *S. aureus* | 85 | 0 | 82 | 76 |
| *E. coli* | 43 | 0 | 38 | 41 |
| *P. aeruginosa* | 61 | 0 | 56 | 58 |
| *B. subtilis* | 28 | 0 | 23 | 26 |
| *C. sporogenes* | 24 | 0 | 19 | 21 |
| *C. albicans* | 16 | 0 | 1 | 18 |
| *A. niger* | 38 | 0 | 42 | 36 |

Die erfindungsgemäßen Kulturmedien lassen sich ohne Probleme gamma-sterilisieren.

## Patentansprüche

1. Gammasterilisierbares Nährmedium auf der Basis von Caseinsojapeptonagar zum Nachweis von Mikroorganismen in wasserstoffperoxidhaltiger Luft oder auf wasserstoffperoxidhaltigen Flächen, **gekennzeichnet durch** Zusätze von 2 - 10 Gew.% Natriumthioglykolat, 5 - 20 Gew.% Natriumthiosulfat und 10 - 30 Gew.% Natriumdisulfit, jeweils bezogen auf das Agar.

2. Gammasterilisierbares Nährmedium nach Anspruch 1, enthaltend 0,1 bis 0,25 % Natriumpyruvat, bezogen auf das Agar.

3. Gammasterilisierbares Nährmedium nach Anspruch 1 oder 2, enthaltend Bromkresolpurpur und Bromkresolviolett als pH-Indikator sowie 10 - 50 Gew.% , vorzugsweise 30 - 45 Gew.%, Polyvinylpyrolidon, bezogen auf das Agar.

4. Gammasterilisierbares Nährmedium nach einem der Ansprüche 1 bis 3, enthaltend 20 - 50% Morpholinopropansulfonsäure und 50 - 80 % Phosphatpuffer, bezogen auf die Gesamtpuffermenge.

5. Gammasterilisierbares Nährmedium nach einem der Ansprüche 1 bis 4, **dadurch gekennzeichnet, dass** als Agar Microbial Content Test Agar eingesetzt wird.

6. Gammasterilisierbares Nährmedium nach einem der Ansprüche 1 bis 5, **dadurch gekennzeichnet, dass** es Betain, Glycin, Cystin, Prolin und Asparagin enthält.

## Claims

1. A gamma-sterilisable nutrient medium on the basis of casein soja peptone agar for the determination of microorganisms in hydrogen peroxide-containing air or on hydrogen peroxide-containing surfaces **characterised by** the addition of from 2 to 10% by weight of sodium thioglycolate, 5 to 20% by weight of sodium thiosulphate and 10 to 30% by weight of sodium disulphite, based on the agar.

2. Gamma-sterilisable nutrient medium according to claim 1 containing from 0.1 to 0.25% of sodium pyruvate, based on the agar.

3. Gamma-sterilisable nutrient medium according to claims 1 or 2 containing bromocresol purple and bromocresol violet as pH-indicator and from 10 to 50% by weight, preferably from 30 to 45% by weight, of polyvinyl pyrrolidone, based on the agar.

4. Gamma-sterilisable nutrient medium according to any of claims 1 to 3 containing from 20 to 50% morpholino propanesulfonit acid and from 50 and 80% phosphate buffer, based on the total amount of buffer.

5. Gamma-sterilisable nutrient medium according to any of claims 1 to 4, **characterised in that** Microbial Content Test Agar is used as agar.

6. A gamma-sterilisable nutrient medium according to any of claims 1 to 5, **characterised in that** it contains betaine, glycine, cysteine, proline, and asparagine.

## Revendications

1. Milieu nutritif stérilisable aux rayons gamma à base gélose de peptone caséine-soja pour la détection de micro-organismes dans une atmosphère contenant du peroxyde d'hydrogène ou sur des flacons contenant du peroxyde d'hydrogène, **caractérisé par** des ajouts de 2 à 10 % en poids de thioglycolate de sodium, de 5 à 20 % en poids de thiosulfate de sodium et de 10 à 30 % en poids de disulfite de sodium, rapportés sur la gélose.

2. Milieu nutritif stérilisable aux rayons gamma selon la revendication 1, contenant 0,1 à 0,25 % en poids de pyruvate de sodium, rapporté sur la gélose.

3. Milieu nutritif stérilisable aux rayons gamma selon la revendication 1 ou 2, contenant du pourpre de bromocrésol et du violet de bromocrésol comme indicateurs de pH ainsi que 10 à 50 % en poids, de préférence 30 à 45 % en poids, de polyvinylpyrrolidone, rapportés sur la gélose.

4. Milieu nutritif stérilisable aux rayons gamma selon l'une des revendications 1 à 3, contenant 20 à 50 % d'acide morpholinopropanesulfonique et 50 à 80 % en poids de tampon phosphate, rapportés sur la quantité totale de tampon.

5. Milieu nutritif stérilisable aux rayons gamma selon l'une des revendications 1 à 4, **caractérisé en ce qu'**est utilisée comme gélose de la gélose Microbial Content Test Agar.

6. Milieu nutritif stérilisable aux rayons gamma selon l'une des revendications 1 à 5, **caractérisé en ce qu'**il contient de la bétaïne, de la glycine, de la cystine, de la proline et de l'asparagine.
